# EUROPÄISCHE PATENTSCHRIFT

(11) **EP 0 546 327 B1**
(45) Veröffentlichungstag und Bekanntmachung des Hinweises auf die Patenterteilung: **17.05.1995**
(21) Anmeldenummer: 92119092.2
(22) Anmeldetag: 07.11.1992
(51) Int. Cl.: A61B 17/00, A61B 19/10

(54) **Hilfsmittel für laparoskopische Operationen**
Accessory for laparoscopic operations
Accessoire pour opérations laparoscopiques

(30) Priorität: 05.12.1991 DE 4140156
(43) Veröffentlichungstag der Anmeldung: 16.06.1993
(73) Patentinhaber: Schaller, Günter, Dr. med., 79110 Freiburg (DE)
(72) Erfinder: Schaller, Günter, W-7800 Freiburg (DE); Klietsch, Dietmar, W-7050 Waiblingen (DE)
(74) Vertreter: KOHLER SCHMID + PARTNER

(56) Entgegenhaltungen:
- DE-A- 3 413 375
- GB-A- 2 199 498
- US-A- 3 286 713
- US-A- 3 736 934
- US-A- 4 953 566
- US-A- 5 037 379

## Beschreibung

Die Erfindung geht aus von Hilfsmitteln für laparoskopische Operationen gemäß dem Oberbegriff des Patentanspruches 1, die einem Kunststoffbehältnis zuordenbar sind, welches zur intraabdominellen, geschützten Zwischenlagerung von laparoskopisch zu entfernenden Gewebeteilen dient.

Derartige Vorrichtungsanschlußelemente sind durch die Veröffentlichung Laparoscopic Nephrectomy: Initial Case Report, The Journal of Urology, Vol. 146, 278 - 282 August 1991, bekanntgeworden.

Bei laparoskopischen Eingriffen, die durch kleine Öffnungen in der Bauchdecke ausgeführt werden, kann die Extraktion von Organen, Organteilen und Steinbildungen schwierig oder ohne weitere Maßnahmen unmöglich und für den Patienten mit zusätzlichen Risiken verbunden sein.

Freipräpariertes, intaktes, nicht-infektiöses Organgewebe wird unter der Operation gewöhnlich im freien Bauchraum gelagert. Ein Risiko ist, das Gewebe nicht wieder aufzufinden. Droht intraabdominelle Keimverschleppung oder Verlust von Organteilen und Steinbildungen, so muß die Extraktion sofort erfolgen und kann den Eingriff komplizieren oder wesentlich verlängern. Ein geeignetes Kunststoffbehältnis, welches intraabdominell plaziert ist, kann zur Aufnahme der zu entfernenden, kontaminierten Gewebeteile gleichsam als Zwischenlager dienen, um vorgenannte Nachteile zu vermeiden.

Die Entfernung von intaktem Organgewebe erfolgt überwiegend durch die Nabelinzision. Unproblematisch ist dies, wenn das Gewebe nach Einziehen in die Trokarhülse mit dieser entfernt wird. Meist ist jedoch der Trokardurchmesser zu gering. Ist dies der Fall, so wird das Gewebe durch die ungeschützte Bauchdecke gezogen und kann diese kontaminieren.

Die während der Extraktion auf das Gewebe wirkenden Zugkräfte führen nicht selten zu Gewebezerstörungen. Dabei gelangen infektiöse Materialen, Gewebeteile, maligne Zellformationen oder Steinbildungen in den freien Bauchraum.

Entzündlich verdicktes Organgewebe oder große Steinbildungen sowie deren Summationseffekte können die Extraktion mechanisch unmöglich machen.

So wurden Techniken und Instrumente entwickelt, mit denen Organgewebe intraabdominell zerkleinert werden kann, z. B. bei Uterusmyomen. Mechanische und Ultraschallithotripsie von Steinbildungen können in der intakten Gallenblase eingesetzt werden, ihre Anwendung im freien Bauchraum für die Zerkleinerung großer Steinbildungen ist nicht möglich.

Bei allen laparoskopischen Methoden muß das Gewebe ab einer bestimmten Größe intraabdominell durch Punktion, Quetschung oder mechanische Zerstörung zur Bergung aus dem Bauchraum zerkleinert werden, da die Vergrößerung der Bauchdeckeninzisionen der Grundidee des laparoskopischen Operierens widerspricht. Durch das intraabdominell zerkleinerte Gewebe nimmt die Infektionsgefahr im Bauchraum zu.

Ist dennoch eine Erweiterung der Bauchdeckeninzisionen bei einem laparoskopischen Eingriff erforderlich, so widerspricht dies ebenfalls der Grundidee des Verfahrens der minimal invasiven Chirurgie, riskiert Blutungen, spätere Hernienbildungen und macht die schichtweise Naht der Bauchdecke erforderlich.

Aus der eingangs genannten Veröffentlichung ist ein Kunststoffsack bekannt, in dem das Resektat noch im Körper der Patienten zerkleinert werden kann. Dazu wurde auch ein spezielles Zerkleinerungsinstrument entwickelt.

Mit dem bekannten Kunststoffsack und den damit eingesetzten Instrumenten ist ein ausreichender Schutz gegen das verfahrensbedingte höhere Kontaminationsrisiko bei laparoskopischen Eingriffen nicht gewährleistet. Bei der Bergung bzw. während der intraabdominellen Zerkleinerung des Gewebes oder der Steinbildungen kann infektiöses oder malignes Material an der Außenseite des Behältnisses in die Bauchdecke und den Bauchraum zurücktropfen.

Weiterhin ist ein großkalibriges Instrument von Klaiber bekannt (Select Sutter GmbH, Katalog Nr. 72 0200, Freiburg, Juni 1991), mit dem große Steine aus dem freien Bauchraum unter Erhalt des Pneumoperitoneums geborgen werden können. Auch bei diesem Instrument kann bei der Bergung des Gewebes infektiöses oder malignes Material aus der Trokarhülse in den Bauchraum zurücktropfen.

DE 34 13 375 beschreibt eine Abdeckvorrichtung für endoskopische Untersuchungen, die besteht aus:
Einer Klemmeinrichtung zur Befestigung eines dehnbaren chirurgischen Abdecktuches an einem starren Endoskoprohr mit einem scheibenförmigen Unterteil, auf den das chirurgische Abdecktuch aufgelegt wird und einem Oberteil, das nach erfolgter Arthroskopie durch seinen offenen Teil auf das Arthroskop zur Fixierung des Abdecktuches geschoben und mit dem Unterteil verschraubt wird und einem flüssigkeitsundurchlässigen, sterilen chirurgischen Loch-Abdecktuch, das zwischen dem Oberteil und Unterteil der Klemmeinrichtung eingeklemmt ist. Das System ist nach entsprechender Sterilisierung mehrfach verwendbar.

US-Patentschrift 5,037,379 beschreibt einen chirurgischen Gewebebeutel für ein perkutanes Entfernen und Zerkleinern von großen, in dem Beutel enthaltenen Mengen an Gewebe. Der Gewebebeutel besteht aus zwei Materialschichten, einer inneren Schicht aus einem durchschlagsicheren Material und einer äußeren Schicht von feuchtigkeitsundurchlässigem Material zur Aufnahme von Zellen und Flüssigkeit. Das Beutelmaterial ist für ein Einführen durch eine Zugangshülle in die Operationsstelle und zum Bilden einer gasdichten Abdichtung, wenn er sich durch die Zugangshülle oder Punktionsstelle erstreckt, faltbar und flexibel. Ein Zugfaden ist am offenen Ende des Beutels befestigt, um den Beutel zu schließen, wenn das Gewebe darin enthalten ist und durch die Punktionsstelle in der Außenfläche der Haupt gezogen wird.

Ausgehend von diesem Stand der Technik (US-A-5 037 379) ist es deshalb Aufgabe der Erfindung, Hilfsmittel für ein bekanntes Kunststoffbehältnis zu schaffen, die es ermöglichen, freipräpariertes Gewebe ohne Gefahr zusätzlicher Kontamination aus dem Bauchraum in toto oder in fragmentierter Form zu bergen und neben der Nabelinzision auch die indirekt betroffenen Bauchdeckeninzisionen vor zusätzlicher Kontamination sicher zu schützen.

Diese Aufgabe wird erfindungsgemäß gemäß den kennzeichnenden Merkmalen des Anspruchs 1 gelöst.

Mit den erfindungsgemäßen Hilfsmitteln ist es möglich, daß eine auf der Bauchdecke liegende Abdeckfolie ausreichender Größe mit dem Kunststoffbehältnis dichtend verbunden werden kann. Neben der durch den Nabeltrokar geschaffenen Bauchdeckeninzision können auch alle anderen, für den Eingriff nötigen Inzisionen gesamthaft vor unerwünschten Kontaminationen geschützt werden. Aus dem Kunststoffbehältnis unkontrolliert austretende Flüssigkeit oder maligne Zellverbände werden sicher von der Abdeckfolie aufgefangen und ein unmittelbarer Kontakt mit der Bauchdecke ist auszuschließen. Werden über geeignete Instrumente Gewebeteile in toto oder in fragmentierter Form dem noch teilweise intraabdominell gelagerten Kunststoffbehältnis entnommen, so ist die Bauchdecke wie auch der Bauchraum vor einem Direktkontakt mit infektiösem oder malignem Gewebe geschützt.

Das im Bauchraum gelagerte Kunststoffbehältnis kann mit freipräpariertem Material befüllt werden und danach wird dieses Behältnis tabaksbeutelartig verschlossen. Das Kunststoffbehältnis weist eine vordefinierte Reißfestigkeit auf, das auch einer schwierigen Extraktion stand hält. Ist das freipräparierte Material oder sind Steinbildungen in das Kunststoffbehältnis eingebracht, so ist der Inhalt verlustsicher und ohne Gefahr vor Keimverschleppung im Bauchraum zwischengelagert.

In einer Weiterbildung der Erfindung ist das Kunststoffbehältnis flexibel und faltbar und weist einen Schaft auf, dessen freies Ende von der Öffnung, die von einem in die Abdeckfolie eingearbeiteten Ring gebildet ist, ummantelt ist, wobei das freie Ende und der Ring mit einem Verschlußelement verbindbar sind. Sind diese Voraussetzungen gegeben, so ist es über Verschlußelemente verschiedenster Formgebung möglich, die Abdeckfolie mit dem Kunststoffbehältnis zu verbinden. Flanschartige und rohrförmige Stopfen können in das außerhalb der Bauchdecke geöffnete Kunststoffbehältnis eingedrückt werden, wobei der oder die Stopfen mit einem Ring der Abdeckfolie verrasten. Dabei umgreift der Ring das Kunststoffbehältnis im Schaftbereich. Das freie Ende des Kunststoffbehältnisses durchgreift den Ring und ein Abschnitt des Schaftes des Kunststoffbehältnisses ist durch das Verrasten der Verbindungsteile zwischen dem Ring und dem Verschlußelement verschiebefest fixierbar.

Vorteilhaft ist es insbesondere, wenn das Verschlußelement aus einem zylinderförmigen Abschnitt mit auf der Außenumfangsfläche ausgebildeten Einkerbungen, insbesondere einer ringförmigen Nut, versehen ist, einen Durchbruch aufweist, und daß der Ring Mittel aufweist, die verrastbar in die Einkerbungen greifen. Der Ring kann dabei zusätzliche Nasen aufweisen, die formschlüssig in Ausnehmungen der ringförmigen Nut eingreifen können.

Dabei ist es besonders vorteilhaft, wenn das Kunststoffbehältnis aus einem durchsichtigen Werkstoff gefertigt ist. Nicht nur die Verrastung zwischen Ring und Verschlußelement kann einfach überprüft werden, sondern auch die Bergung des freipräparierten Materials aus dem Kunststoffbehältnis kann unter optischer Kontrolle erfolgen.

Weist das Kunststoffbehältnis entfaltet einen kugelförmigen Abschnitt und einen zylinderförmigen Abschnitt auf, so ist es sowohl für die Aufnahme des frei präparierten Gewebes wie für die Aufnahme von Instrumenten geeignet, die über die Öffnung in das Kunststoffbehältnis eingreifen. Zur Extraktion wird der Nabeltrokar entfernt und das Kunststoffbehältnis wird sofort in die Nabelinzision gezogen und dichtet diese ab, so daß das Pneumoperitoneum erhalten bleibt. Der kugelförmige Abschnitt des Kunststoffbehältnisses ist dabei vollkommen intraabdominell gelagert und der zylinderförmige Abschnitt des Kunststoffbehältnisses ragt teilweise aus der Bauchdecke heraus. Über den freiliegenden Abschnitt des Kunststoffbehältnisses wird dann der Ring der Abdeckfolie geschoben und in das Behältnis wird das Verschlußelement eingedrückt, das dann mit dem Ring verrastet. Das Kunststoffbehältnis ist dadurch verschiebefest fixiert. Das Anlegen eines gegenüber dem Pneumoperitoneum erhöhten Druckes in dem Kunststoffbehältnis führt zu dessen intraabdominellen Entfaltung. Eventuelle Manipulationen, wie z.B. Zerkleinerung des Resektats, können auf diese Art und Weise von außen her bequem im Inneren des Kunststoffbehältnisses unter Videosicht vorgenommen werden.

Für besondere Anwendungen ist es auch denkbar, daß mehrere Kunststoffbehältnisse voneinander trennbar in einem Kunststoffbehältnis vorgesehen sind. Dies erlaubt intraabdominell eine getrennte Lagerung verschiedenster Gewebeverbände, die nach der Extraktion wieder einander zugeordnet bzw. wieder getrennt untersucht werden können.

Ist im Bereich des verschließ- und öffenbaren Endes des Kunststoffbehältnisses ein farblich deutlich unterscheidbares Band, das so die Öffnung des Beutels markiert, als zusammenziehbare Schlaufe in das Kunststoffbehältnis eingearbeitet, so stellt diese gegenständliche Ausgestaltung eine einfache und eine besonders sichere Form dar, wie das Kunststoffbehältnis verschlossen und wieder geöffnet werden kann.

Die Abdeckfolie ist aus einem hautverträglichen Material gefertigt und sie weist im Bereich der Öffnung eine verstärkte Materialdicke gegenüber der flächenhaft ausgebildeten Abdeckfolie auf. Die Öffnung in der Abdeckfolie wird dadurch geschaffen, daß beispielsweise Material der Abdeckfolie zur Bildung eines Rings verwendet wird oder in ein Loch der Abdeckfolie wird ein Ring eingesetzt, der mit der Abdeckfolie materialschlüssig verbunden wird.

In einer besonders vorteilhaften Ausbildung der Erfindung greift der zylinderförmige Abschnitt durch die Bauchdecke hindurch. Über den zylinderförmigen Abschnitt wird die Nabelinzision entgegen der Rückstellkräfte der Bauchdecke offen gehalten und wird in den Durchbruch des zylinderförmigen Abschnitts ein Hohlzylinder formschlüssig und dichtend eingeführt, so ist es möglich, sofern der Hohlzylinder im Bereich des freien Endes eine abdichtbare Öffnung und mehrere zum Lumen des Hohlzylinders hin durchgängige rohrförmige Ansätze aufweist, daß das Innenvolumen des Kunststoffbehältnisses gespült werden kann, und gleichzeitig können Instrumente zur Lithotripsie in das Innere des Kunststoffbehältnisses eingeführt werden. Ist dabei das Kunststoffbehältnis aus einen durchsichtigen Material gefertigt, so kann die Lithotripsie unter optischer Kontrolle durchgeführt werden.

Das im Kunststoffbehältnis gelagerte Gewebe kann unter intraabdomineller Sicht in einem geschlossenen, spülbaren Raum zerkleinert werden. Nach Abschluß der Gewebezerkleinerung können die Hilfsmittel in toto entfernt und entsorgt werden. Die Hilfsmittel eignen sich insbesondere zum Einsatz bei der laparoskopischen Cholezystektomie.

Weitere Vorteile ergeben sich aus der Beschreibung der beigefügten Zeichnung. Ebenso können die vorstehend genannten und die noch weiter aufgeführten Merkmale erfindungsgemäß jeweils einzeln oder in beliebigen Kombinationen miteinander verwendet werden. Die erwähnten AuSführungsformen sind nicht als abschließende Aufzählung zu verstehen, sondern haben vielmehr beispielhaften Charakter.

Die Erfindung ist in der Zeichnung dargestellt und wird anhand von Ausführungsbeispielen näher erläutert. Es zeigen:
- Fig. 1: erfindungsgemäße Hilfsmittel, teilweise im Schnitt, wobei ein Kunststoffbehältnis teilweise intraabdominell gelagert ist;
- Fig. 2: eine Explosionsdarstellung der erfindungsgemäßen Hilfsmittel;
- Fig. 3: einen Schnitt durch einen Hohlzylinder, wie er in ein Verschlußelement dichtend einführbar ist;
- Fig. 4: ein erfindungsgemäßes Kunststoffbehältnis mit mehreren im Kunststoffbehältnis gelagerten weiteren Kunststoffbehältnissen;
- Fig. 5: eine vergrößerte Darstellung von erfindungsgemäßen Hilfsmitteln im Schnitt, wobei das Verschlußelement durch die Bauchwand hindurchgreift.

Die einzelnen Figuren der Zeichnung zeigen den erfindungsgemäßen Gegenstand teilweise stark schematisiert und sind nicht maßstäblich zu verstehen. Die Gegenstände der einzelnen Figuren sind teilweise vergrößert dargestellt, damit ihr Aufbau besser gezeigt werden kann.

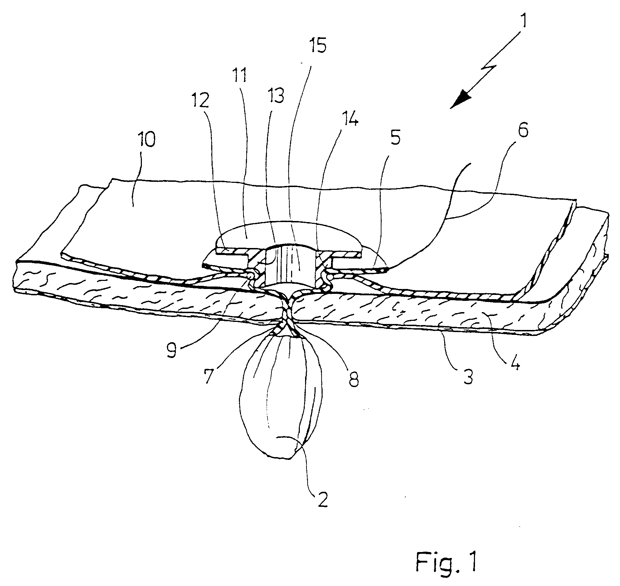

Fig. 1 zeigt mit 1 Elemente, wie sie bei laparoskopischen Operationen Verwendung finden. Ein Kunststoffbehältnis 2 ist durch das Peritoneum 3 und die Bauchwand 4 nach außen hindurchgeführt. Im Bereich eines freien Endes 5 ist in das Kunststoffbehältnis 2 ein Band 6 eingearbeitet, das als zusammenziehbare Schlaufe ausgebildet ist. Mittels dieser Schlaufe läßt sich das aus einer stabilen und reißfesten sowie flexiblen und faltbaren Kunststoffolie gebildete Kunststoffbehältnis 2 durch das Zusammenziehen des Bandes 6 schließen. Wird das Band 6 gelockert, so läßt sich das freie Ende 5 des Kunststoffbehältnisses 2 öffnen. Natürliche Rückstellkräfte der Bauchdeckeninzision verschließen das in der Figur dargestellte Kunststoffbehältnis 2 dicht. Ein Schaft 8 des Kunststoffbehältnisses 2 ist außerhalb der Bauchwand 4 von einem Ring 9 vollkommen ummantelt, der materialschlüssig mit einer Abdeckfolie 10 verbunden ist. Ein Verschlußelement 11, das einstückig aus einem ringförmig bis scheibenförmigen Abschnitt 12 und einem zylinderförmigen Abschnitt 13 gebildet ist, greift mit dem zylinderförmigen Abschnitt 13 in das Kunststoffbehältnis 2. Über eine am Außenmantel des zylinderförmigen Abschnitts 13 ausgebildete ringförmige Nut 14 ist der Ring 9 mit dem Verschlußelement 11 verrastet. Die Kontur des Ringes 9 ist derart an die ringförmige Nut 14 angepaßt, daß der Schaft 8 des Kunststoffbehältnisses 2 zwischen dem zylinderförmigen Abschnitt 13 und dem Ring 9 verlaufen kann und der Ring 9 dennoch kraft- und formschlüssig in der Nut 14 verrastet. Das Verschlußelement 11 weist einen Durchbruch 15 auf, der in der in Fig. 1 gezeigten Form im Querschnitt beispielhaft kreisförmig ist. Das Verschlußelement 11 liegt mit einem Ende indirekt über das Kunststoffbehältnis 2 auf der Haut auf. Mit geeigneten Instrumenten können die Rückstellkräfte der Bauchdeckeninzision überwunden werden und aus dem im wesentlichen intraabdominell angeordneten Kunststoffbehältnis 2 kann das im Kunststoffbehältnis 2 enthaltene Gewebe oder Steinbildungen geborgen werden. Das Verschlußelement 11 kann auch derart angeordnet sein, daß es mit seinem zylinderförmigen Abschnitt 13 die Bauchwand 4 und das Peritoneum 3 durchgreift. Die in der Fig. 1 gezeigte Verrastverbindung zwischen dem Ring 9 und dem Verschlußelement 11 stellt in beispielhafter Form eine flüssigkeitsdichte Verbindung dar und schützt die Bauchwand 4 und die Inzision sicher vor Kontamination.

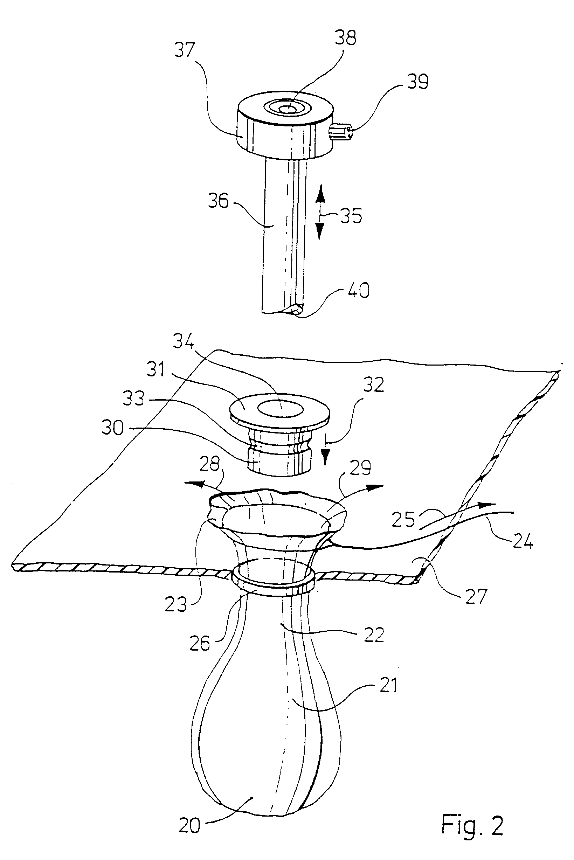

Fig. 2 zeigt einzelne Elemente der Erfindung in einer Explosionsdarstellung. Das Kunststoffbehältnis 20 ist geöffnet dargestellt. Auf die Zuordnung der einzelnen Elemente zur Bauchwand wurde in dieser Figur verzichtet. Ein Kunststoffbehältnis 20 ist aus einem durchsichtigen flexiblen Material hergestellt. Zusätzlich können in das Material zur Erhöhung der Reißfestigkeit Fasern eingearbeitet sein. In der Figur ist das flexible und faltbare Kunststoffbehältnis 20 entfaltet. Es setzt sich aus einem kugelförmigen Abschnitt 21 und einem zylinderförmigen Abschnitt 22 zusammen. Im Bereich des freien Endes 23 ist in den zylinderförmigen Abschnitt 22 ein Band 24 eingearbeitet, das, sofern in Pfeilrichtung 25 gezogen wird, den zylinderförmigen Abschnitt 22 sicher verschließt.
Der zylinderförmige Abschnitt 22 des Kunststoffbehältnisses 20 ist von einem Ring 26 beabstandet umgriffen, der Bestandteil einer Abdeckfolie 27 ist. Der Ring 26 ist fest mit der Abdeckfolie 27 verbunden. Der Ring 26 weist eine größere Materialdicke als die Abdeckfolie 27 auf. In Pfeilrichtungen 28, 29 kann das freie Ende 23 des zylinderförmigen Abschnittes 22 derart geweitet werden, daß ein hohlzylinderförmiger Abschnitt 30 eines Verschlußelementes 31 in Pfeilrichtung 32 in den zylinderförmigen Abschnitt 22 des Kunststoffbehältnisses 20 hineinverschoben werden kann. Das Verschlußelement 31 ist derart auf den Ring 26 abgestimmt, daß der Ring 26 über Einkerbungen 33 mit dem hohlzylinderförmigen Abschnitt 30 lösbar verrasten kann. Im verrasteten Zustand ist dann der zylinderförmige Abschnitt 22 des Kunststoffbehältnisses 20 zwischen dem Verschlußelement 31 und dem Ring 26 verschiebefest und unverrückbar angeordnet. In einem Durchbruch 34 des Verschlußelementes 31 läßt sich ein hohlzylinderförmiges Rohr 36 in Pfeilrichtungen 35 flüssigkeitsdicht verschieben. Das Rohr 36 weist am freien Ende 37 eine mit einer Dichtung versehene Öffnung 38 auf. Zusätzlich ist an dem freien Ende 37 ein rohrförmiger Ansatz 39 vorgesehen, der mit einem Lumen 40 des Rohres 36 in Verbindung steht. Das Rohr 36 dient als Instrumentenschaft für Instrumente zur Gewebe- und/oder Steinzertrümmerung. Der Ansatz 39 kann als Spül- und/oder Absaugkanal eingesetzt werden. Auch ein weiterer Kanal kann am Rohr 36 vorgesehen sein, um über einen erhöhten Gasdruck das Kunststoffbehältnis 20 intraabdominell zu entfalten.

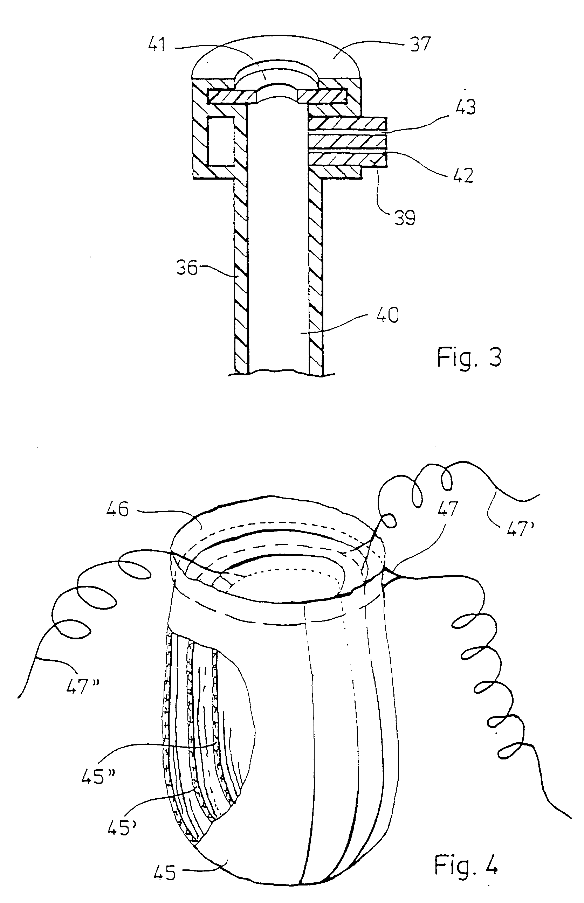

Fig. 3 zeigt das Rohr 36 im Schnitt, wie es beispielsweise, wie unter Fig. 2 beschrieben, eingesetzt wird. Das Rohr 36 weist am freien Ende 37 eine muffenförmige Ausbildung auf, die eine Dichtung 41, zur Abdichtung der eingeführten Instrumente, aufnimmt. Durch den Ansatz 39 sind die Kanäle 42, 43 hindurchgeführt. Die Kanäle 42, 43 stehen mit dem Lumen 40 in Verbindung. Der Außendurchmesser des Rohres 36 ist an den Durchbruch des jeweiligen Verschlußelementes angepaßt.

Fig. 4 zeigt stark schematisiert ein erfindungsgemäßes Kunststoffbehältnis 45, das ausgefaltet eine weitgehend zylinderförmige Form aufweist. Das Kunststoffbehältnis 45 ist aus einer hochreißfesten Folie gefertigt, die faserverstärkte Elemente aufweist. Im Bereich einer Öffnung 46 ist in das Material des Kunststoffbehältnisses 45 ein Band 47 eingearbeitet, mit dem sich das Kunststoffbehältnis 45 schließen läßt. Im Kunststoffbehältnis 45 können je nach Bedarf weitere Kunststoffbehältnisse 45', 45'' gelagert sein, die intraabdominell aus dem Kunststoffbehältnis 45 herausgenommen werden können. Über das Band 47 bzw. Band 47', 47'' ist das jeweilige Kunststoffbehältnis 45, 45', 45'' von außen manipulierbar.

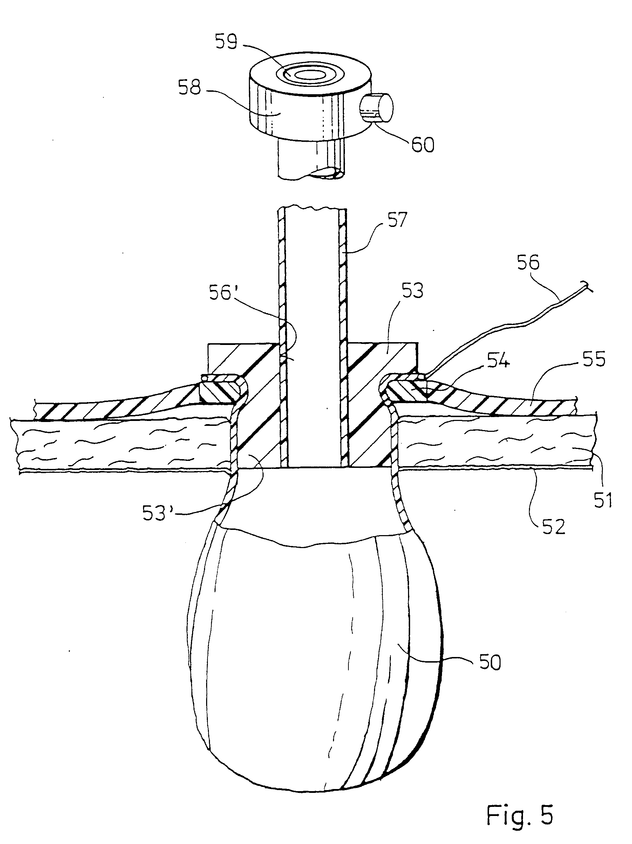

Fig. 5 zeigt ein Kunststoffbehältnis 50, teilweise im Schnitt, wie es teilweise intraabdominell gelagert ist. Der Schaft des Kunststoffbehältnisses 50 durchgreift eine Bauchwand 51 und das Peritoneum 52. Ein Verschlußelement 53, das flansch- und pfropfenartig ausgebildet ist, greift in das Kunststoffbehältnis 50 ein, durchgreift mit einem zylinderförmigen Abschnitt 53' die Bauchwand 51 und das Peritoneum 52 und ist mit einem Ring 54 einer Abdeckfolie 55 verrastet. Mit 56 ist ein Band dargestellt, über das das Kunststoffbehältnis 50 tabaksbeutelartig verschlossen und wieder geöffnet werden kann. Über die Verrastung des Verschlußelements 53 mit dem Ring 54 ist das Kunststoffbehältnis 50 verschiebefest und dichtend mit der Abdeckfolie 55 verbunden. In einen Durchbruch 56' des Verschlußelementes 53 ist ein Hohlzylinder 57 dichtend einführbar, der an seinem freien Ende eine Muffe 58 aufweist, die geeignet ist, Zertrümmerungsgeräte dichtend zu halten und im Hohlzylinder 57 lagestabil zu führen. Eine Dichtung ist mit 59 in der Figur eingezeichnet. Die Dichtung 59 umgreift beispielsweise ein Lithotripsiegerät dichtend, so daß der Hohlzylinder 57 und das Innenvolumen des Kunststoffbehältnisses 50 über einen Ansatz 60 gasbefüllt und/oder gespült werden kann. Über den Ansatz 60 ist auch eine Absaugung möglich bzw. eine Gaseinführung, über die das Kunststoffbehältnis 50 intraabdominell entfaltet werden kann.

Wie die Figur 5 zeigt, ist das Verschlußelement 53 durch die Bauchwand hindurchgeführt und drückt die Außenwand des Kunststoffbehältnisses 50 dichtend gegen die Innenumfangsfläche der Nabelinzision. Das Pneumoperitoneum bleibt erhalten.

## Patentansprüche

1. Hilfsmittel für laparoskopische Operationen, aufweisend ein Kunststoffbehältnis, welches zur intraabdominellen, geschützten Zwischenlagerung von laparoskopisch zu entfernenden Gewebeteilen dient, dadurch gekennzeichnet, daß die Hilfsmittel (1) auch eine mit einer Öffnung versehene Abdeckfolie (10, 27, 55) und ein Verschlußelement (11) aufweisen, daß das Kunststoffbehältnis (2; 20; 45; 45'; 45''; 50) ein verschließ- und öffenbares Ende (5) aufweist, und daß im Gebrauchszustand das Ende (5) außerhalb der Bauchwand mittels des Verschlußelements (11) dichtend an der Öffnung der Abdeckfolie (10, 27, 55) gekoppelt ist.

2. Hilfsmittel nach Anspruch 1, dadurch gekennzeichnet, daß das Kunststoffbehältnis (2) flexibel und faltbar ist und einen Schaft (8) aufweist, dessen freies Ende (5) von der Öffnung, die von einem in die Abdeckfolie (10) eingearbeiteten Ring (9) gebildet ist, ummantelt ist, wobei das freie Ende (5) und der Ring (9) im Gebrauchszustand des Hilfsmittels mittels des Verschlußelements (11) miteinander verbunden sind.

3. Hilfsmittel nach Anspruch 2, dadurch gekennzeichnet, daß das freie Ende (5) den Ring (9) durchgreift und daß im Gebrauchszustand des Hilfsmittels ein Abschnitt des Schaftes (8) zwischen dem Ring (9) und dem Verschlußelement (11) verschiebefest fixiert ist.

4. Hilfsmittel nach Anspruch 2 oder 3, dadurch gekennzeichnet, daß das Verschlußelement (11) einen zylinderförmigen Abschnitt (13) mit auf der Außenumfangsfläche ausgebildeten Einkerbungen aufweist, insbesondere eine ringförmige Nut (14), und daß der Abschnitt (13) mit einem Durchbruch versehen ist, wobei der Ring (9) Mittel aufweist, die verrastbar in die Einkerbungen greifen.

5. Hilfsmittel nach einem der Ansprüche 1 bis 4, dadurch gekennzeichnet, daß das Kunststoffbehältnis (2; 20; 45; 45'; 45''; 50) aus einem durchsichtigen Material gefertigt ist.

6. Hilfsmittel nach Anspruch 5, dadurch gekennzeichnet, daß das Kunststoffbehältnis (20) entfaltet einen kugelförmigen Abschnitt (21) und einen zylinderförmigen Abschnitt (22) aufweist.

7. Hilfsmittel nach einem der Ansprüche 1 bis 6, dadurch gekennzeichnet, daß mehrere Kunststoffbehältnisse (45'; 45'') voneinander trennbar in einem Kunststoffbehältnis (45) vorgesehen sind.

8. Hilfsmittel nach einem der Ansprüche 1 bis 7, dadurch gekennzeichnet, daß im Bereich des verschließ- und öffenbaren Endes (5; 23) ein farblich deutlich unterscheidbares Band (6; 24) als zusammenziehbare Schlaufe in das Kunststoffbehältnis (2; 20) eingearbeitet ist.

9. Hilfsmittel nach einem der Ansprüche 1 bis 8, dadurch gekennzeichnet, daß die Abdeckfolie (10; 27; 55) aus einem hautverträglichen Material gefertigt ist, und daß die in der Materialdicke gegenüber der Abdeckfolie (10; 27; 55) verstärkte Öffnung Mittel aufweist, die mit der Abdeckfolie (10; 27; 55) einen Materialverbund bilden.

10. Hilfsmittel nach Anspruch 4, dadurch gekennzeichnet, daß der zylinderförmige Abschnitt (13; 22; 53') so dimensioniert ist, daß er im Gebrauchszustand durch die Bauchdecke (4; 51) hindurchgreift.

11. Hilfsmittel nach Anspruch 4 oder 10, dadurch gekennzeichnet, daß der Durchbruch (15; 34; 56') einem Außendurchmesser eines Hohlzylinders (36; 54) formschlüssig und dichtend angepaßt ist.

12. Hilfsmittel nach Anspruch 11, dadurch gekennzeichnet, daß der Hohlzylinder (36) im Bereich des freien Endes (37) eine abdichtbare Öffnung und mehrere zum Lumen (40) des Hohlzylinders (36) hin durchgängige rohrförmige Ansätze (39, 42, 43) aufweist.

## Claims

1. Accessory device for laparoscopic operations comprising a plastic receptacle which serves for the safe intermediate storage in the lower abdomen, of parts of tissue to be removed laparoscopically, characterized in that the accessory means (1) comprise also a cover foil (10, 27, 55) having an opening, and a closure element (11), the plastic receptacle (2; 20; 45; 45'; 45''; 50) comprises an end (5) which can be closed and opened and, when the accessory device is in use, the end (5) is coupled at the opening of the cover foil (10, 27, 55) outside the abdominal wall by means of the closure element (11) in a sealing manner.

2. Accessory device according to claim 1, characterized in that the plastic receptacle (2) is flexible and foldable, and comprises a shaft (8) whose free end (5) is enclosed by an opening formed in the cover sheet (10) by an integrated ring (9), wherein the free end (5) and the ring (9) are connected to one another by means of the closure element (11) when the accessory device is in use.

3. Accessory device according to claim 2, characterized in that the free end (5) extends through the ring (9), and, when the accessory device is in use, a portion of the shaft (8) is fixed against displacement between the ring (9) and the closure element (11).

4. Accessory device according to claim 2 or 3, characterized in that the closure element (11) is provided with a cylindrical section (13) with notches, in particular an annular groove (14), formed on its outer circumferential surface, and the section (13) is provided with an opening, the ring (9) being provided with means that can be brought into locking engagement with the notches.

5. Accessory device according to any of claims 1 to 4, characterized in that the plastic receptacle (2; 20; 45; 45'; 45''; 50) is made from a transparent material.

6. Accessory device according to claim 5, characterized in that, in the unfolded condition, the plastic receptacle (20) comprises a spherical section (21) and a cylindrical section (22).

7. Accessory device according to any of claims 1 to 6, characterized in that a plurality of separable plastic receptacles (45'; 45'') are arranged in one plastic receptacle (45).

8. Accessory device according to any of claims 1 to 7, characterized in that a cord (6; 24) of an easily distinguishable colour is integrated in the plastic receptacle (2; 20), in the form of a loop that can be pulled together, in the area of that end (5; 23) which can be opened and closed.

9. Accessory device according to any of claims 1 to 8, characterized in that the cover sheet (10; 27; 55) is made from a material compatible with human skin, and the opening which exhibits a greater material thickness than the remaining areas of the cover sheet (10; 27; 55) comprises means forming a material compound with the cover sheet (10; 27; 55).

10. Accessory device according to claim 4, characterized in that the cylindrical section (13; 22; 53') is dimensioned such that it extends through the abdominal wall (4; 51) when being used.

11. Accessory device according to claim 4 or 10, characterized in that the opening (15; 34; 56') is adapted to an outer diameter of a hollow cylinder (36; 54) in a form-locking and sealing manner.

12. Accessory device according to claim 11, characterized in that the hollow cylinder (36) is equipped, in the area of its free end (37) with a sealable opening and a plurality of continuous tubular connection pieces (39, 42, 43) opening into the lumen (40) of the hollow cylinder (36).

## Revendications

1. Accessoires pour opérations laparoscopiques, présentant un réservoir en matière synthétique qui sert à un stockage transitoire protégé, intra-abdominal, de morceaux de tissu à éliminer par voie laparoscopique, caractérisé en ce que les accessoires (1) présentent également une feuille de recouvrement (10, 27, 55) pourvue d'une ouverture et un élément de fermeture (11), en ce que le réservoir en matière synthétique (2 ; 20 ; 45 ; 45' ; 45'' ; 50) présente une extrémité (5) qui peut être ouverte et fermée et en ce que, à l'état d'utilisation, l'extrémité (5) est couplée de manière étanche à l'ouverture de la feuille de recouvrement (10, 27, 55) à l'extérieur de la paroi du ventre, au moyen de l'élément de fermeture (11).

2. Accessoire suivant la revendication 1, caractérisé en ce que le réservoir en matière synthétique (2) est flexible et pliable et en ce qu'il présente un tronc (8) dont l'extrémité libre (5) est enveloppée par l'ouverture qui est formée par un anneau (9) incorporé dans la feuille de recouvrement (10), l'extrémité libre (5) et l'anneau (9) étant mutuellement reliés au moyen de l'élément de fermeture (11) à l'état d'utilisation de l'accessoire.

3. Accessoire suivant la revendication 2, caractérisé en ce que l'extrémité libre (5) passe à travers l'anneau (9) et en ce que, à l'état d'utilisation de l'accessoire, une section du tronc (8) est fixée entre l'anneau (9) et l'élément de fermeture (11) de manière à ne pas pouvoir coulisser.

4. Accessoire suivant l'une des revendications 2 et 3, caractérisé en ce que l'élément de fermeture (11) présente une section cylindrique (13) avec des entailles réalisées sur la surface périphérique extérieure, en particulier une rainure annulaire (14), et en ce que la section (13) est pourvue d'un perçage, l'anneau (9) présentant des moyens qui pénètrent dans les entailles de manière à pouvoir s'y encliqueter.

5. Accessoire suivant l'une des revendications 1 à 4, caractérisé en ce que le réservoir en matière synthétique (2 ; 20 ; 45 ; 45' ; 45'' ; 50) est fabriqué en une matière transparente.

6. Accessoire suivant la revendication 5, caractérisé en ce que le réservoir en matière synthétique (20) déployé présente une section de forme sphérique (21) et une section de forme cylindrique (22).

7. Accessoire suivant l'une des revendications 1 à 6, caractérisé en ce que plusieurs réservoirs en matière synthétique (45' ; 45'') sont prévus de manière séparable l'un de l'autre dans un réservoir en matière synthétique (45).

8. Accessoire suivant l'une des revendications 1 à 7, caractérisé en ce que, dans la zone de l'extrémité (5 ; 23) qui peut être ouverte et fermée, une bande nettement différenciable par la couleur (6 ; 24) est incorporée dans le réservoir en matière synthétique (2 ; 20) sous la forme d'un noeud coulant.

9. Accessoire suivant l'une des revendications 1 à 8, caractérisé en ce que la feuille de recouvrement (10 ; 27 ; 55) est fabriquée en une matière compatible pour la peau et en ce que l'ouverture renforcée dans l'épaisseur de matière par rapport à la feuille de recouvrement (10 ; 27 ; 55) présente des moyens qui forment un composite avec la feuille de recouvrement (10 ; 27 ; 55).

10. Accessoire suivant la revendication 4, caractérisé en ce que la section cylindrique (13 ; 22 ; 53') est dimensionnée de façon que, à l'état d'utilisation, elle passe à travers la paroi du ventre (4 ; 51).

11. Accessoire suivant l'une des revendications 4 et 10, caractérisé en ce que le perçage (15 ; 34 ; 56') est adapté de manière étanche et de façon à former une liaison par la forme à un diamètre extérieur d'un cylindre creux (34 ; 54).

12. Accessoire suivant la revendication 11, caractérisé en ce que le cylindre creux (36) présente, dans la zone de l'extrémité libre (37), une ouverture étanchéifiable et plusieurs saillies tubulaires (39, 42, 43) en communication vers la lumière (40) du cylindre creux (36).
